# EUROPEAN PATENT SPECIFICATION

(11) **EP 4 354 138 B1**
(45) Date of publication and mention of the grant of the patent: **12.08.2026**
(21) Application number: 22818184.8
(22) Date of filing: 12.05.2022
(51) Int. Cl.: G01N 33/48, G01N 33/552, G01N 1/30, G01N 33/52

(54) **CONTROL SLIDE GLASS FOR PATHOLOGICAL EXAMINATION AND METHOD FOR MANUFACTURING SAME**
KONTROLLSCHIEBERGLAS ZUR PATHOLOGISCHEN UNTERSUCHUNG UND VERFAHREN ZUR HERSTELLUNG DAVON
VERRE POUR LAMES DE CONTRÔLE D'EXAMEN PATHOLOGIQUE ET SON PROCÉDÉ DE FABRICATION

(30) Priority: 07.06.2021 JP 2021095160
(43) Date of publication of application: 17.04.2024
(73) Proprietor: HITACHI HIGH-TECH CORPORATION, Tokyo 105-6409 (JP)
(72) Inventor: SASAKI Taira, Tokyo 100-8280 (JP); MARUYAMA Masashi, Tokyo 100-8280 (JP); SAKUMA Hirotaka, Tokyo 100-8280 (JP); SAKURAI Toshinari, Tokyo 105-6409 (JP); MATSUBARA Shigeki, Tokyo 105-6409 (JP)
(74) Representative: Mewburn Ellis LLP
(86) International application number: PCT/JP2022/020043
(87) International publication number: WO 2022/259810

(56) References cited:
- WO-A2-2009/085576
- CN-A- 103 116 018
- JP-A- 2006 519 376
- JP-A- 2020 523 614
- JP-A- S60 237 365
- US-A1- 2016 123 850
- US-A1- 2016 274 006
- US-A1- 2020 116 601
- RIERA JOSE, JEAN F. SIMPSON, ROSALBA TAMAYO, HECTOR BATTIFORA: "Use of Cultured Cells as a Control for Quantitative Immunocytochemical Analysis of Estrogen Receptor in Breast Cancer: The Quicgel Method", AMERICAN JOURNAL OF CLINICAL PATHOLOGY, AMERICAN SOCIETY FOR CLINICAL PATHOLOGY, US, vol. 111, no. 3, 1 March 1999 (1999-03-01), US , pages 329 - 335, XP093014726, ISSN: 0002-9173, DOI: 10.1093/ajcp/111.3.329

## Description

### Technical Field

The present invention relates to a control glass slide for a pathological examination and a method for manufacturing the same.

### Background Art

A pathological examination is a method of diagnosing the presence or absence of a lesion or the type of the lesion by preparing a specimen preparation in which cells or tissues collected from a patient are attached to a glass slide and performing observation, for example, observation at a microscopic level. In a pathological examination, a staining method for components of cells or tissues is essential to detect a lesion from colorless cells or tissues.

One of the staining methods used for a pathological examination is, for example, immunostaining. In immunostaining, a specific antigen expressed in cells or tissues is detected with an antibody reagent. Since localization of an antigen to be examined can be visualized, immunostaining is a very important staining method in a pathological examination.

Since immunostaining is generally often used for a definitive diagnosis, it is necessary to determine whether an appropriate staining result is obtained. In order to obtain reliability of the staining result in a medical setting, it is strongly recommended to perform control staining of a control known to be stained in advance together with staining of a specimen.

As a glass slide capable of simultaneously staining a specimen and a control substance, a glass slide into which a control substance is introduced has been proposed (see, for example, PTLs 1 and 2).

### Citation List

### Patent Literatures

PTL 1: WO 2016/143717 A
PTL 2: WO 2018/181483 A
PTL 3: US2016274006A1

### Summary of Invention

### Technical Problem

In each of the glass slides into which a control substance is introduced proposed in PTLs 1 and **2,** the control substance is immobilized on the outermost surface, and therefore, there has been a possibility that a dye generated from the control substance at the time of immunostaining flows out to a specimen or the stability of the control substance is low.

Therefore, an object of the present disclosure is to provide a control glass slide containing a control substance in a form in which the control glass slide is capable of preventing outflow of a dye generated by staining of the control substance for the purpose of accuracy control in a pathological examination.

Another object of the present disclosure is to provide a method for manufacturing a control glass slide capable of achieving the aforementioned object.

### Solution to Problem

In a first aspect, the present invention relates to a control glass slide for a pathological examination, including:
a control spot on a glass slide, the control spot containing a control substance in the pathological examination and a dye trapping material. The control glass slide of the present invention is defined in claim 1.

In a second aspect, the present invention relates to a method for manufacturing the control glass slide, the method including the steps of:
preparing a mixture containing a control substance and a dye trapping material; and
forming a control spot containing the mixture on a glass slide.

In a third aspect, the present invention relates to an alternative method for manufacturing the control glass slide, the method including the steps of:
fixing a control substance onto a glass slide; and
forming a control spot on the glass slide by applying a dye trapping material onto the control substance fixed onto the glass slide. The methods of the present invention are defined in claims 12 and 14, respectively.

### Advantageous Effects of Invention

According to the present disclosure, it is possible to provide a control glass slide capable of simultaneously staining a specimen and a control substance on one glass slide, and to provide a method for manufacturing a control glass slide. The control glass slide of the present disclosure can prevent outflow of a dye generated by staining of a control substance.

### Brief Description of Drawings

[FIG. 1] FIG. 1 is a schematic cross-sectional view showing one aspect of a control glass slide of the present disclosure.
[FIG. 2] FIG. 2 is a schematic cross-sectional view showing one aspect of a control glass slide of the present disclosure.
[FIG. 3] FIG. 3 shows a staining result of a control glass slide tested in Example 3.
[FIG. 4] FIG. 4 shows a staining result of a control glass slide tested in Example 4.
[FIG. 5] FIG. 5 shows a staining result of a glass slide tested in Comparative Example 1.
[FIG. 6] FIG. 6 shows a staining result of a glass slide tested in Comparative Example 2.

### Description of Embodiments

One aspect of the present invention relates to a control glass slide for a pathological examination, including a control spot on a glass slide, the control spot containing a control substance in the pathological examination and a dye trapping material.

According to the aforementioned aspect, it is possible to provide a control glass slide capable of simultaneously staining a specimen and a control substance on one glass slide. The control glass slide means a glass slide having a control spot and a portion for mounting and staining a specimen preparation. The control glass slide of the aforementioned aspect can prevent outflow of a dye generated by staining of a control substance.

One aspect of the present invention relates to a method for manufacturing the control glass slide, including the steps of preparing a mixture containing a control substance and a dye trapping material, and forming a control spot containing the mixture on a glass slide.

Further, one aspect of the invention relates to a method for manufacturing the control glass slide, including the steps of fixing a control substance onto a glass slide, and forming a control spot on the glass slide by applying a dye trapping material onto the control substance fixed onto the glass slide.

According to these aspects, the control glass slide can be manufactured.

Hereinafter, the present invention will be described in detail.

### [Control glass slide]

The control glass slide according to the present invention is a control glass slide for a pathological examination, and includes a control spot on a glass slide, the control spot containing a control substance in the pathological examination and a dye trapping material. Since the control glass slide according to the present invention contains the dye trapping material in the control spot, it is possible to prevent outflow of the dye from the control spot at the time of staining. The control glass slide is usually used for an examination involving staining such as immunostaining among pathological examinations. The provision of the control glass slide according to the present invention improves the diagnostic accuracy of a staining method in a pathological examination. In addition, since the control substance is included in a form in which the control glass slide is capable of preventing outflow of a staining dye, it is possible to prevent the occurrence of false positives caused by external outflow of the staining dye.

The control glass slide according to the present invention is used with a specimen preparation mounted thereon. Simultaneous staining of the specimen preparation and the control spot with the specimen preparation mounted thereon can stain the specimen and the control substance under the same conditions. For example, in an enzyme-labeled antibody method in immunostaining, a specific antigen is detected using an antibody. In the staining method, it is generally necessary for a multistep normal reaction, such as an antigen-antibody reaction between a primary antibody and an enzyme-labeled antibody, and a subsequent enzymatic reaction of a chromogenic dye, to proceed. The multistep normal reaction is, for example, a reaction in which a primary antibody specifically binds to a specific antigen, an enzyme-labeled antibody specifically binds to the primary antibody, and an enzymatic reaction of a chromogenic dye proceeds.

Use of the control glass slide according to the present invention enables investigation of the progress of the aforementioned multistep normal reaction. Use of the control glass slide in, for example, an automatic staining device for a pathological examination enables investigation of whether the automatic staining device is normally activated.

In general, it is ideal that a control glass slide for pathological staining has the following three characteristics, but a control glass slide that satisfies all of the characteristics has not yet been realized.
(1) A specimen and a control substance are simultaneously stained. Immunostaining is performed by sequential adding, mixing, heating, and the like of a plurality of staining reagents, and it is necessary to perform a control examination for investigating that there is no abnormality in each staining reagent, that each step has been appropriately performed, and the like under the same conditions.
(2) A dye does not flow out to the outside during staining. In a design in which a specimen and a control substance are simultaneously stained, when a dye flows out from the control spot to the outside at the time of staining the control substance, the specimen that is not supposed to be stained may be stained with the out-flowing dye (false positive may occur).
(3) The storage stability of a control substance is high. It is desirable that the control glass slide is stored until use, but a control substance introduced onto the glass slide is dried and oxidized in a state of being in contact with the outside air, and a proper staining result may not be obtained.

The control glass slide according to the present invention has the aforementioned characteristics (1) to (3), and therefore is very useful as a control glass slide for a pathological examination.

The control glass slide according to the present invention includes the control spot on the glass slide, and therefore, the control glass slide can perform the control examination simultaneously with the examination of the specimen under the same conditions. The control glass slide according to the present embodiment contains the dye trapping material together with the control substance in the control spot, and therefore, it is possible to prevent outflow of the dye from the control spot to the outside at the time of staining the control substance. The control substance is present together with the dye trapping material in the control spot, and therefore, it is considered that the contact of the control substance with the outside air is reduced as compared with the case where the control substance is immobilized on the outermost surface, and drying and oxidation can be prevented.

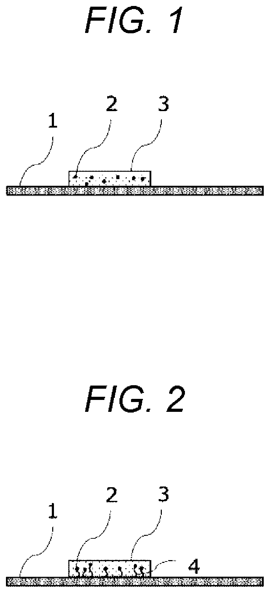

Configuration examples of the glass slide according to the present embodiment include one aspect shown in FIG. 1 and one aspect shown in FIG. 2.

In the aspect shown in FIG. 1 (also referred to as a first aspect), a control spot containing a control substance 2 and a dye trapping material 3 is present on a glass slide 1. In the first aspect, the control substance 2 is embedded in the dye trapping material 3, and is preferably physically immobilized on the dye trapping material 3.

Also in the aspect shown in FIG. 2 (also referred to as a second aspect), a control spot containing a control substance 2 and a dye trapping material 3 is present on a glass slide 1. In the second aspect, the control substance 2 is immobilized on the glass slide 1 with a polyfunctional linker molecule 4. The control substance 2 is usually embedded in the dye trapping material 3.

In the control glass slide of the first aspect, it is preferable that the control substance is embedded and immobilized in the dye trapping material. The control glass slide of the first aspect can be manufactured, for example, by a method in which a control substance is mixed in a dye trapping material in a solution or sol state, and then the dye trapping material is solidified. The control substance can be introduced onto the glass slide as a control spot by being immobilized on the dye trapping material. Examples of the method for solidifying the dye trapping material include a method by chemical crosslink formation using a chemical crosslinking agent, a method by polymer formation using a polymerization initiator, and a method by physical network formation using an ionic additive.

In the control glass slide of the second aspect, it is preferable that the control substance is immobilized on the glass slide and embedded in the dye trapping material. The control glass slide of the second aspect can be manufactured, for example, by a method in which a control substance is immobilized on a glass slide and then covered with a dye trapping material in a solution or sol state, and then the dye trapping material is solidified. In this case, the control substance is introduced onto the glass slide by being immobilized on the glass slide. As a method for immobilizing the control substance on the glass slide, an immobilization method using a polyfunctional linker molecule can be applied. Examples of the method for solidifying the dye trapping material include a method by chemical crosslink formation using a chemical crosslinking agent, a method by polymer formation using a polymerization initiator, and a method by physical network formation using an ionic additive in the same manner as in the first aspect.

The control substance is, for example, a substance suspected to be expressed in a specimen, and is a substance intended for detection in a pathological examination. In addition, another example of the control substance is a substance that exhibits a behavior similar to that of a substance intended for detection in a pathological examination. Examples of the substance that exhibits a similar behavior in a pathological examination include a part of a substance intended for detection or a substance having such a part, or a modified material of a substance intended for detection.

It is one preferable aspect that the control substance contains at least one substance selected from a protein, a peptide, an amino acid oligomer, and a nucleic acid. Since these substances are often suspected to be expressed in a specimen (substances intended for detection) in a pathological examination, it is desirable to use these substances as control substances according to the type of pathological examination.

For example, in immunostaining, a marker protein for a specific disease can be used as a control substance.

The control glass slide is preferably used for accuracy control of immunostaining among staining methods performed in a pathological examination. In this case, the control substance preferably contains a protein, a peptide, or an amino acid oligomer. Specifically, in particular, a marker protein for a specific disease or a peptide having a sequence partially identical to that of a marker protein for a specific disease is preferable. Examples of the marker protein for a specific disease include Ki-67, p53, CK, D2-40, CD20, CD3, ER, PGR, HER2, CD34, CD79, CD10, αSMA, S100, CD68, CK7, BCL2, CD56, p63, CD5, CK20, Synaptophysin, Vimentin, Chromogranin A, TTF-1, Desmin, CK-HMW, CD31, EMA, CD117, Cyclin D1, CD30, Myeloperoxidase, CD4, and CEA.

In the present disclosure, a protein, a peptide, and an amino acid oligomer are not strictly distinguished, but for example, one in which 50 or more amino acids are peptide-linked may be referred to as a protein, one in which 11 to 49 amino acids are peptide-linked may be referred to as a peptide, and one in which 2 to 10 amino acids are peptide-linked may be referred to as an amino acid oligomer.

The control glass slide may be applied to accuracy control of a staining examination other than immunostaining. Examples of the staining examination other than immunostaining include in situ hybridization (ISH) for detecting a specific nucleic acid (DNA or RNA) in cells or tissues. In this case, a nucleic acid (for example, DNA, RNA, or a fragment thereof) can be used as the control substance.

The dye trapping material is not particularly limited as long as it can prevent outflow of the dye from the control spot to the outside at the time of staining the control substance. The dye trapping material desirably has permeability to a staining reagent, has low non-specific adsorption to a staining reagent, and has high transparency.

A marker protein for a specific disease is stained because of having an antibody recognition site, and thus can be used as a control substance as described above. However, when the marker protein is left in contact with the outside air for a long period of time, the marker protein is dried and oxidized to denature the antibody recognition site, and a normal staining result may not be obtained. In the control glass slide according to the present disclosure, the control substance such as a specific disease marker protein is embedded in the dye trapping material, so that drying and oxidation are prevented, and the control glass slide has long-term storage stability.

For example, in immunostaining, diaminobenzidine (DAB) is generally used as a chromogenic dye. DAB is polymerized by peroxidase generated by an enzyme-labeled antibody to become polymer DAB (pDAB), and is deposited as an insoluble precipitate, thereby staining the control substance or the surrounding of the control substance. At this time, when there is no dye trapping material, pDAB generated by the control substance reaches a specimen present on the same glass slide, so that a normal staining result may not be obtained. In contrast, in the glass slide for accuracy control according to the present invention since the dye trapping material is included, pDAB is generated inside the dye trapping material after the staining reagent or DAB penetrates to the dye trapping material, so that outflow of pDAB to the outside can be prevented, and a normal staining result can be obtained. In addition, since the control glass slide according to the present invention has the control spot on the glass slide and the control spot contains the dye trapping material, drying of the control substance can be prevented, and long-term storage can be expected.

It is one preferable aspect that the dye trapping material contains at least one material selected from an amino acid polymer, a sugar polymer, and a synthetic polymer. One of the materials or two or more of the materials may be used. The dye trapping material is preferably made of such a material because the dye trapping material is easily immobilized on the glass slide. The molecular weight of the dye trapping material is not particularly limited, but is usually 10 kDa to 1,000 kDa.

The amino acid polymer preferably contains at least one polymer selected from gelatin, collagen, albumin, polyglutamic acid, and polylysine. The dye trapping material preferably contains such an amino acid polymer because it is easy to solidify by utilizing the functional group of the polymer.

The sugar polymer preferably contains at least one polymer selected from agarose, alginic acid, hyaluronic acid, carrageenan, pectin, methyl cellulose, and hydroxyethyl cellulose. The dye trapping material preferably contains such a sugar polymer because it is easy to solidify by utilizing a functional group of the polymer.

The synthetic polymer preferably contains at least one polymer selected from an acrylic acid-based polymer, a methacrylic acid-based polymer, an acrylamide-based polymer, a methacrylamide-based polymer, and an ethylene glycol-based polymer. The dye trapping material preferably contains such a synthetic polymer because it is easy to solidify by polymer formation using a polymerization initiator.

As the dye trapping material, gelatin and collagen are particularly preferable because gelatin and collagen exhibit a temperature-dependent solid-liquid phase change, and therefore can be molded only by being allowed to stand at a predetermined temperature after being mixed with the control substance.

It is one preferable aspect that the dye trapping material is solidified for the purpose of immobilizing the dye trapping material on the glass slide. That is, in the control glass slide according to the present embodiment, it is one preferable aspect that the dye trapping material is solidified. Examples of the solidification method include a method by chemical crosslink formation using a chemical crosslinking agent, a method by polymer formation using a polymerization initiator, and a method by physical network formation using an ionic additive.

Examples of the chemical crosslinking agent include at least one chemical crosslinking agent selected from DMT-MM (4-(4,6-dimethoxy-1,3,5-triazin-2-yl)-4-methylmorpholinium chloride), formaldehyde, glutaraldehyde, paraformaldehyde, dimethyl suberimidate, genipin, (benzotriazol-1-yloxy)tris-(dimethylamino)phosphonium, benzotriazol-1-yloxytris(pyrrolidino)phosphonium hexafluorophosphate, a polyfunctional ethylene glycol-based polymer, and a carbodiimide compound.

The molecular weight of the polyfunctional ethylene glycol-based polymer is not particularly limited, and for example, poly(ethylene glycol) diamine, poly(ethylene glycol) di(3-aminopropyl), poly(ethylene glycol) diglycidyl ether, or poly(ethylene glycol) di(hydroxysuccinimide) can be used.

As the carbodiimide compound, for example, diisopropylcarbodiimide, dicyclohexylcarbodiimide, 1-ethyl-3-(3-dimethylaminopropyl)carbodiimide hydrochloride, or 1-cyclohexyl-3-(2-morpholinoethyl)carbodiimide metho-p-toluenesulfonate can be used.

As the polymerization initiator, for example, an azo-based polymerization initiator such as 2,2'-azobis-2-amidinopropane dihydrochloride or azobisisobutyronitrile, or a persulfate such as sodium persulfate, potassium persulfate, or ammonium persulfate can be used.

As the ionic additive, for example, a calcium salt such as calcium chloride, calcium hydrogen phosphate, calcium dihydrogen phosphate, calcium trihydrogen phosphate, calcium phosphate, calcium carbonate, calcium sulfate, or calcium lactate can be used.

In the second aspect, the control substance is fixed onto the glass slide with a polyfunctional linker molecule. The polyfunctional linker molecule preferably contains at least one linker molecule selected from glutaraldehyde, a silane coupling agent, and a polyfunctional ethylene glycol-based polymer. These linker molecules can easily fix the control substance onto the glass slide, and therefore are preferable. The polyfunctional linker molecule may be a bifunctional linker molecule or a linker molecule having three or more functional groups.

As the silane coupling agent, for example, 3-aminopropyltrimethoxysilane, 3-aminopropyltriethoxysilane, N-2-(aminoethyl)-3-aminopropylmethyldimethoxysilane, N-2-(aminoethyl)-3-aminopropyltrimethyldimethoxysilane, 3-isocyanatopropyltriethoxysilane, 3-trimethoxysilylpropylsuccinic anhydride, 3-(3,4-epoxycyclohexyl)ethyltrimethoxysilane, 3-glycidoxypropylmethyldimethoxysilane, 3-glycidoxypropyltrimethoxysilane, 3-glycidoxypropylmethyldiethoxysilane, or 3-glycidoxypropyltriethoxysilane can be used.

One preferable aspect of the polyfunctional ethylene glycol-based polymer is a bifunctional ethylene glycol-based polymer. The molecular weight of the polyfunctional ethylene glycol-based polymer is not particularly limited, and for example, poly(ethylene glycol) diamine, poly(ethylene glycol) di(3-aminopropyl), poly(ethylene glycol) diglycidyl ether, or poly(ethylene glycol) di(hydroxysuccinimide) can be used.

The control glass slide according to the present invention contains the control substance and the dye trapping material in the control spot, and may contain the aforementioned components and reactants thereof or the like. In addition, the control spot may contain a solvent used in the process of forming the control spot, for example, water, an alcohol, or the like.

In the control glass slide according to the present invention it is one preferable aspect that the control spot has an area of 1 mm² or more and 1,000 mm² or less. The area of the control spot is more preferably 4 mm² or more and 200 mm² or less. The area of the control spot is preferably within the aforementioned range because observation at the time of performing a pathological examination is easy and productivity is high.

The control glass slide according to the present invention may be a control glass slide having one control spot or a control glass slide having a plurality of control spots. The number of control spots can be appropriately determined according to the type and purpose of the pathological examination.

The control glass slide according to the present invention may be a microarray-type control glass slide having a plurality of control spots. In the case of a microarray-type control glass slide in which a plurality of control spots having different types of control substances are placed on a glass slide, it is not necessary to manufacture a control glass slide for each specific control substance. Further, in immunostaining, the microarray-type control glass slide can also be used as a control when a cocktail antibody containing a plurality of types of antibodies is used as a staining reagent, and it is also possible to check whether an examination of a plurality of cases is normally completed in one assay.

In the microarray-type control glass slide, a control spot having a control substance not intended for examination can be provided as a negative control spot. Providing the negative control spot enables to accurately know that an error in an operation has occurred when an error in a staining operation has occurred such that all specimens are stained. When the control glass slide has a plurality of control spots, as the dye trapping materials and the control substances that form the plurality of control spots, those different between the control spots may be adopted.

A control glass slide having a plurality of control spots, for example, a microarray-type control glass slide preferably has an area per control spot of 1 mm² or more and 200 mm² or less, and more preferably 4 mm² or more and 50 mm² or less. The area of the control spot is preferably within the aforementioned range because observation at the time of performing a pathological examination is easy and productivity is high.

In embodiments the control spot has a thickness of 0.1 µm or more and 1,000 µm or less. In particular, in the aforementioned first aspect, it is only necessary for the staining reagent to penetrate to a part of the dye trapping material, and therefore, from the viewpoint that a tissue section actually used for immunostaining has a size of about 5 µm, and from the viewpoint of the purpose of trapping the dye and productivity, the thickness of the control glass slide is preferably 0.1 µm or more and 1,000 µm or less, and more preferably 1 µm or more and 100 µm or less.

In some embodiments the thickness of the control spot is 0.1 µm or more and 200 µm or less. In particular, in an aspect in which the control substance is fixed onto the glass slide with a polyfunctional linker molecule, for example, in the aforementioned second aspect, it is necessary for the staining reagent to penetrate to the bottom where the control substance is fixed. Therefore, the thickness of the control spot is preferably 0.1 µm or more and 200 µm or less, and more preferably 1 µm or more and 20 µm or less.

The form and shape of the control spot of the control glass slide according to the present embodiment are not particularly limited, but it is preferable that the control spot is in a porous form, a swollen gel form, or a xerogel form since it is easy to accurately perform the pathological examination. It is particularly preferable that the control spot is in a porous form because highly sensitive staining can be expected due to a high washing effect when staining is performed. Examples of the method for making the control spot porous include a method described in Examples (a method including a dehydration step with ethanol). The control spot in a swollen gel form can be obtained, for example, by using an acrylic acid-based polymer as the dye trapping material in the presence of a solvent. The control spot in a xerogel form can be obtained, for example, by freeze-drying the control spot in a swollen gel form.

The ratio between the control substance and the dye trapping material in the control spot of the glass slide according to the present embodiment is not particularly limited. When considering the staining strength at the time of staining and the production cost, the mass ratio of the dye trapping material to the control substance is preferably 1 : 0.00001 to 1 : 0.1, and more preferably 1 : 0.0001 to 1 : 0.01.

The amount of the control substance per unit area of the control spot of the control glass slide according to the present embodiment is preferably 0.01 to 1,000 ng/mm² and more preferably 0.1 to 100 ng/mm² in order to carry out sufficient staining.

In some embodiments the amount of the control substance contained in the control spot can be quantified by using a fluorescent reagent. For example, in immunostaining, a marker protein for a specific disease can be used as the control substance. In this case, a marker protein introduced into the control spot can be quantified by quantifying a fluorescence intensity using a fluorescently labeled IgG exhibiting specific binding to a marker protein for a specific disease. This method can be used as a quality assurance method for the control glass slide. At this time, it is possible to perform a total examination of the control glass slide by selecting fluorescently labeled IgG that does not affect immunostaining. The type of fluorescent reagent can be appropriately changed according to the type of control substance. The control substance bound to such a fluorescent reagent is also referred to as a fluorescently labeled control substance. That is, the control glass slide according to the present embodiment can contain a fluorescently labeled control substance as the control substance.

### [Method for manufacturing control glass slide]

The method for manufacturing a control glass slide according to the present invention is a method for manufacturing the aforementioned control glass slide, and there are roughly two aspects.

A first aspect is a method for manufacturing the control glass slide, including the steps of preparing a mixture containing a control substance and a dye trapping material, and forming a control spot containing the mixture on a glass slide. In the aspect, the control glass slide of the aforementioned first aspect can be manufactured.

In the first aspect, it is one preferable aspect that the mixture is solidified into a tubular shape, and the method includes the step of forming a control spot on a glass slide by cutting the mixture into a thin film form and attaching the mixture in the thin film form to the glass slide.

A second aspect is a method for manufacturing the control glass slide, including the steps of fixing a control substance onto a glass slide, and forming a control spot on the glass slide by applying a dye trapping material onto the control substance fixed onto the glass slide. In the aspect, the control glass slide of the aforementioned second aspect can be manufactured using a polyfunctional linker molecule when the control substance is fixed onto the glass slide.

Specific examples of the first aspect include a method in which a mixed liquid of gelatin and a control substance is added to a mold made of Teflon (registered trademark), covered with a glass slide, and solidified under refrigeration, and the dye trapping material is transferred onto the glass slide to form a control spot formed from the dye trapping material and the control substance on the glass slide.

In the first aspect, for example, as a method for manufacturing the control glass slide in a large amount, it is possible to adopt a method in which the dye trapping material and the control substance are enclosed and mixed in a pipe-like tubular mold, then, the dye trapping material is solidified into a tubular shape to obtain a mixture solidified into a tubular shape, and the mixture is cut into a thin film form and attached to the glass slide.

Specific examples of the second aspect include a method in which the control substance is immobilized on the glass slide with glutaraldehyde, and then, collagen is applied thereto to form the control spot formed from the dye trapping material and the control substance on the glass slide.

Also when the microarray-type control glass slide is manufactured, the method for manufacturing the control glass slide in a large amount described above can be adopted. For example, it is possible to manufacture the microarray-type control glass slide in a large amount by enclosing and mixing different types of control substances individually in a plurality of dye trapping materials, then solidifying the dye trapping materials into a tubular shape to obtain a plurality of mixtures solidified into a tubular shape, bundling the plurality of mixtures, cutting the mixtures into a thin film form, and attaching the mixtures to the glass slide.

In some embodiments a solvent such as water or an alcohol may be used for the purpose of adjusting the viscosity of the dye trapping material or the mixture of the control substance and the dye trapping material. All or a part of the solvent used may remain in the control spot, or the solvent used may be removed by drying or the like.

The method for manufacturing the control glass slide according to the present embodiment is not limited to the above examples, and a manufacturing method according to a purpose can be adopted.

### Examples

Hereinafter, the present embodiment will be specifically described by way of examples. However, the present embodiment is not limited to the following examples.

### [Example 1]

In Example 1, a control glass slide of the first aspect was produced.

Recombinant CD20 was used as the control substance, and gelatin and BSA were used as the dye trapping material. Recombinant CD20, gelatin, BSA, and water were mixed and kept at 37°C to prepare a mixed aqueous solution containing 5 wt% gelatin, 2.5 wt% BSA, and 100 µg/mL recombinant CD20 (balance: water).

The mixed aqueous solution (20 µL) was taken and added to a Teflon mold, and then covered with a glass slide, and allowed to stand in a refrigerator for 1 hour. After the glass slide was removed from the mold and the transfer of the dye trapping material containing the control substance onto the glass slide was verified, the dye trapping material was solidified by immersing the glass slide in a 2 wt% DMT-MM/ethanol solution at 25°C for 5 hours. Subsequently, unreacted DMT-MM residues were blocked by immersing the glass slide in 4% ethanolamine/ethanol at 25°C for 12 hours. The control glass slide having the control spot containing the control substance and the dye trapping material was produced by washing the glass slide.

The control spot produced by this method was in a porous form, the control spot had an area of 240 mm² and a thickness of 20 µm, the amount of the antigen contained per unit area of the control spot was 8 ng/mm², and the amount of the dye trapping material per unit area was 4 µg/mm².

### [Example 2]

In Example 2, a control glass slide of the second aspect was produced.

Recombinant CD20 was used as the control substance, gelatin and BSA were used as the dye trapping material, and glutaraldehyde was used as a bifunctional linker molecule. Recombinant CD20 and a glutaraldehyde aqueous solution were mixed to prepare a mixed aqueous solution containing 100 µg/mL recombinant CD20 and 1 wt% glutaraldehyde (balance: water), and the mixed aqueous solution was allowed to stand at 37°C for 1 hour. The mixed aqueous solution was added onto an amino-coated glass slide and allowed to stand for 1 day to obtain a glass slide on which recombinant CD20 was immobilized.

Subsequently, a mixed aqueous solution containing 5 wt% gelatin and 2.5 wt% BSA (balance: water) was prepared, 20 µL of the mixed aqueous solution was taken and added to a Teflon mold, and then covered with the glass slide on which recombinant CD20 was immobilized, and allowed to stand in a refrigerator for 1 hour. After the glass slide was removed from the mold and the transfer of the dye trapping material onto the glass slide was verified, the dye trapping material was solidified by immersing the glass slide in a 2 wt% DMT-MM/ethanol solution at 25°C for 5 hours. Subsequently, unreacted DMT-MM residues were blocked by immersing the glass slide in 4% ethanolamine/ethanol at 25°C for 12 hours. The control glass slide having the control spot containing the control substance and the dye trapping material was produced by washing the glass slide.

The control spot produced by this method was in a porous form, the control spot had an area of 240 mm² and a thickness of 20 µm, the amount of the antigen contained per unit area of the control spot was 4 ng/mm², and the amount of the dye trapping material per unit area was 4 µg/mm².

### [Example 3]

In Example 3, immunostaining was performed using the control glass slide produced in Example 1.

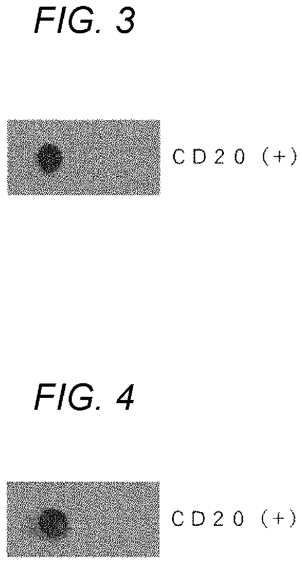

To the control glass slide produced in Example 1, 100 µL of 50 µg/mL anti-CD20 mouse monoclonal antibody was added to cause a reaction at 37°C for 16 minutes. After the glass slide was washed, 100 µL of 50 µg/mL peroxidase-labeled antimouse IgG goat polyclonal antibody was added thereto to cause a reaction at 37°C for 8 minutes. After the glass slide was washed, 100 µL of a 3,3-diaminobenzidine solution and 100 µL of a hydrogen peroxide solution were added thereto to cause a reaction at 37°C for 8 minutes. After the glass slide was washed, 100 µL of an aqueous copper sulfate solution was added thereto to cause a reaction at 37°C for 4 minutes. The glass slide was washed, and the stained state was investigated. It was verified that the control substance was stained. In addition, no outflow of the dye was observed. The control glass slide after staining is shown in FIG. 3.

### [Example 4]

In Example 4, immunostaining was performed using the control glass slide produced in Example 2.

To the control glass slide produced in Example 2, 100 µL of 50 µg/mL anti-CD20 mouse monoclonal antibody was added to cause a reaction at 37°C for 16 minutes. After the glass slide was washed, 100 µL of 50 µg/mL peroxidase-labeled antimouse IgG goat polyclonal antibody was added thereto to cause a reaction at 37°C for 8 minutes. After the glass slide was washed, 100 µL of a 3,3-diaminobenzidine solution and 100 µL of a hydrogen peroxide solution were added thereto to cause a reaction at 37°C for 8 minutes. After the glass slide was washed, 100 µL of an aqueous copper sulfate solution was added thereto to cause a reaction at 37°C for 4 minutes. The glass slide was washed, and the stained state was investigated. It was verified that the control substance was stained. In addition, no outflow of the dye was observed. The control glass slide after staining is shown in FIG. 4.

### [Example 5]

In Example 5, the control glass slide of the first aspect was produced in a large amount.

Recombinant p53 was used as the control substance, and gelatin and BSA were used as the dye trapping material. Recombinant p53, gelatin, BSA, and water were mixed and kept at 37°C to prepare a mixed aqueous solution (10 mL) containing 5 wt% gelatin, 2.5 wt% BSA, and 100 µg/mL recombinant p53 (balance: water).

The mixed aqueous solution was poured into a pipe-like Teflon mold (length: 30 mm, outer diameter: 20 mm, inner diameter: 10 mm), and allowed to stand in a refrigerator for 24 hours. The resultant was removed from the mold, and then immersed in a 2 wt% DMT-MM/ethanol solution at 25°C for 24 hours to solidify the dye trapping material. Subsequently, unreacted DMT-MM residues were blocked by immersing the resultant in 4% ethanolamine/ethanol at 25°C for 24 hours. The produced mixture of the dye trapping material and the control substance was cut to a thickness of 10 µm with a microtome and mounted on a glass slide to produce 25 control glass slides at a time.

The control spot produced by this method was in a porous form, the control spot had an area of 300 mm² and a thickness of 10 µm, the amount of the antigen contained per unit area of the control spot was 6 ng/mm², and the amount of the dye trapping material per unit area was 3 µg/mm².

### [Comparative Example 1]

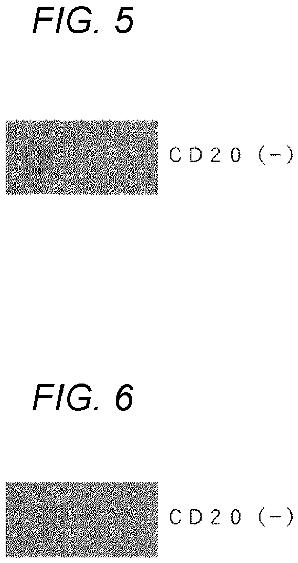

In Comparative Example 1, a glass slide was produced in the same manner as in Example 1 except that recombinant CD20 as the control substance was not used, and immunostaining was performed in the same manner as in Example 3. Staining was not observed. The glass slide after staining is shown in FIG. 5.

### [Comparative Example 2]

In Comparative Example 2, a glass slide was produced in the same manner as in Example 2 except that recombinant CD20 as the control substance was not used, and immunostaining was performed in the same manner as in Example 4. Staining was not observed. The glass slide after staining is shown in FIG. 6.

### [Comparative Example 3]

In Comparative Example 3, a control substance was fixed onto a glass slide using a bifunctional linker molecule without using a dye trapping material, and immunostaining was performed using the obtained glass slide.

In Comparative Example 3, recombinant CD20 was used as the control substance, and glutaraldehyde was used as the bifunctional linker. Recombinant CD20 and a glutaraldehyde aqueous solution were mixed to prepare a mixed aqueous solution containing 100 µg/mL recombinant CD20 and 1 wt% glutaraldehyde (balance: water), and the mixed aqueous solution was allowed to stand at 37°C for 1 hour. The mixed aqueous solution was added onto an amino-coated glass slide and allowed to stand for 1 day to obtain a glass slide on which recombinant CD20 was immobilized.

Immunostaining was performed in the same manner as in Example 4 except that the glass slide on which recombinant CD20 was immobilized was used. Although staining of the control substance was observed, 20% of the colored dye was deposited on the control substance or its periphery, and 80% thereof flowed out to the outside. The deposited amount and the outflow amount were calculated by collecting all the out-flowing liquid and measuring the absorbance of the deposited portion and the out-flowing liquid to estimate the concentration of the dye.

From the examples and the comparative examples, it was suggested that the control glass slide of the examples enables easy staining without flowing out of the dye, and is useful in accuracy control in a pathological examination.

Upper limits and/or lower limits of numerical ranges described in the present description can be arbitrarily combined to specify a preferable range. For example, an upper limit and a lower limit of a numerical range can be arbitrarily combined to specify a preferable range, upper limits of numerical ranges can be arbitrarily combined to specify a preferable range, or lower limits of numerical ranges can be arbitrarily combined to specify a preferable range.

Claims following this written disclosure are hereby expressly incorporated into this written disclosure, with each claim standing on its own as a separate embodiment. The present disclosure includes all permutations of the independent claims with their dependent claims. Further, additional embodiments derived from the independent claims and the subsequent dependent claims are also expressly incorporated into this written description.

It should be understood that throughout the present description, a singular expression includes the concept of plural form thereof unless otherwise stated. Thus, it should be understood that a singular article (for example, "a", "an, "the", or the like in English) also includes the concept of plural form thereof unless otherwise stated. Hereinafter, supplementary notes are further disclosed regarding the present invention.

### Reference Signs List

- 1: glass slide
- 2: control substance
- 3: dye trapping material
- 4: polyfunctional linker molecule

## Claims

1. A control glass slide for a pathological examination, comprising:
a control spot on a glass slide, the control spot containing a control substance in the pathological examination and a dye trapping material,
wherein the control substance is fixed onto the glass slide with a polyfunctional linker molecule,
and wherein the polyfunctional linker molecule contains at least one linker molecule selected from glutaraldehyde, a silane coupling agent, and a polyfunctional ethylene glycol-based polymer.

2. The control glass slide according to claim 1, wherein the control substance contains at least one substance selected from a protein, a peptide, an amino acid oligomer, and a nucleic acid.

3. The control glass slide according to claim 1, wherein the dye trapping material contains at least one material selected from an amino acid polymer, a sugar polymer, and a synthetic polymer.

4. The control glass slide according to claim 3, wherein
the amino acid polymer contains at least one polymer selected from gelatin, collagen, albumin, polyglutamic acid, and polylysine,
the sugar polymer contains at least one polymer selected from agarose, alginic acid, hyaluronic acid, carrageenan, pectin, methyl cellulose, and hydroxyethyl cellulose, and
the synthetic polymer contains at least one polymer selected from an acrylic acid-based polymer, a methacrylic acid-based polymer, an acrylamide-based polymer, a methacrylamide-based polymer, and an ethylene glycol-based polymer.

5. The control glass slide according to claim 1, wherein the control spot has an area of 1 mm² or more and 1,000 mm² or less.

6. The control glass slide according to claim 1, wherein the control spot has a thickness of 0.1 µm or more and 1,000 µm or less.

7. The control glass slide according to claim 1, wherein the control spot has a thickness of 0.1 µm or more and 200 µm or less.

8. The control glass slide according to claim 1, comprising a plurality of control spots.

9. The control glass slide according to claim 8, wherein an area per control spot is 1 mm² or more and 200 mm² or less.

10. The control glass slide according to claim 1, wherein the control substance contains a fluorescently labeled control substance.

11. The control glass slide according to any one of claims 1 to 10, wherein the control spot is in a porous form, a swollen gel form, or a xerogel form.

12. A method for manufacturing the control glass slide according to claim 1, the method comprising the steps of:
preparing a mixture containing a control substance and a dye trapping material; and
forming a control spot containing the mixture on a glass slide.

13. The method according to claim 12, wherein
the mixture is solidified into a tubular shape, and
the method comprises the step of forming a control spot on a glass slide by cutting the mixture into a thin film form and attaching the mixture in the thin film form to the glass slide.

14. A method for manufacturing the control glass slide according to claim 1, the method comprising the steps of:
fixing a control substance onto a glass slide; and
forming a control spot on the glass slide by applying a dye trapping material onto the control substance fixed onto the glass slide.

## Patentansprüche

1. Kontrollobjektträger für eine pathologische Untersuchung, der Folgendes umfasst:
einen Kontrollpunkt auf einem Objektträger, wobei der Kontrollpunkt eine Kontrollsubstanz in der pathologischen Untersuchung und ein Farbstoff-einfangendes Material enthält,
wobei die Kontrollsubstanz mit einem polyfunktionellen Linker-Molekül auf dem Objektträger fixiert ist und
wobei das polyfunktionelle Linker-Molekül zumindest ein Linker-Molekül enthält, das aus Glutaraldehyd, einem Silan-Kupplungsmittel und einem polyfunktionellen Polymer auf Ethylenglykol-Basis ausgewählt ist.

2. Kontrollobjektträger nach Anspruch 1, wobei die Kontrollsubstanz zumindest eine Substanz enthält, die aus einem Protein, einem Peptid, einem Aminosäure-Oligomer und einer Nukleinsäure ausgewählt ist.

3. Kontrollobjektträger nach Anspruch 1, wobei das Farbstoff-einfangende Material zumindest ein Material enthält, das aus einem Aminosäure-Polymer, einem Zucker-Polymer und einem synthetischen Polymer ausgewählt ist.

4. Kontrollobjektträger nach Anspruch 3, wobei das Aminosäure-Polymer zumindest ein Polymer enthält, das aus Gelatine, Collagen, Albumin, Polyglutaminsäure und Polylysin ausgewählt ist,
wobei das Zuckerpolymer zumindest ein Polymer enthält, das aus Agarose, Alginsäure, Hyaluronsäure, Carrageenan, Pektin, Methylcellulose und Hydroxyethylcellulose ausgewählt ist, und
das synthetische Polymer zumindest ein Polymer enthält, das aus einem Polymer auf Acrylsäure-Basis, einem Polymer auf Methacrylsäure-Basis, einem Polymer auf Acrylamid-Basis, einem Polymer auf Methacrylamid-Basis und einem Polymer auf Ethylenglykol-Basis ausgewählt ist.

5. Kontrollobjektträger nach Anspruch 1, wobei der Kontrollpunkt eine Fläche von 1 mm² oder mehr und 1.000 mm² oder weniger aufweist.

6. Kontrollobjektträger nach Anspruch 1, wobei der Kontrollpunkt eine Dicke von 0,1 µm oder mehr und 1.000 µm oder weniger aufweist.

7. Kontrollobjektträger nach Anspruch 1, wobei der Kontrollpunkt eine Dicke von 0,1 µm oder mehr und 200 µm oder weniger aufweist.

8. Kontrollobjektträger nach Anspruch 1, der eine Vielzahl von Kontrollpunkten umfasst.

9. Kontrollobjektträger nach Anspruch 8, wobei die Fläche pro Kontrollpunkt 1 mm² oder mehr und 200 mm² oder weniger beträgt.

10. Kontrollobjektträger nach Anspruch 1, wobei die Kontrollsubstanz eine fluoreszierend markierte Kontrollsubstanz enthält.

11. Kontrollobjektträger nach einem der Ansprüche 1 bis 10, wobei der Kontrollpunkt in poröser Form, in Form eines gequollenen Gels oder in Xerogel-Form vorliegt.

12. Verfahren zur Herstellung eines Kontrollobjektträgers nach Anspruch 1, wobei das Verfahren die folgenden Schritte umfasst:
das Herstellen eines Gemischs, das eine Kontrollsubstanz und ein Farbstoff-einfangendes Material enthält; und
das Ausbilden eines Kontrollpunkts, der das Gemisch enthält, auf einem Objektträger.

13. Verfahren nach Anspruch 12, wobei:
das Gemisch zu einer rohrförmigen Form verfestigt wird und
das Verfahren den Schritt des Ausbildens eines Kontrollpunkts auf einem Objektträger durch Schneiden des Gemischs zu einem dünnen Film und Befestigen des Gemischs in Form des dünnen Films auf dem Objektträger umfasst.

14. Verfahren zur Herstellung eines Kontrollobjektträgers nach Anspruch 1, wobei das Verfahren die folgenden Schritte umfasst:
das Fixieren einer Kontrollsubstanz auf einem Objektträger; und
das Ausbilden eines Kontrollpunkts auf dem Objektträger durch Aufbringen eines Farbstoff-einfangenden Materials auf die auf dem Objektträger fixierte Kontrollsubstanz.

## Revendications

1. Lame de verre de contrôle pour un examen pathologique, comprenant :
un point de contrôle sur une lame de verre, le point de contrôle contenant une substance de contrôle dans l'examen pathologique et un matériau de piégeage de colorant,
dans laquelle la substance de contrôle est fixée sur la lame de verre avec une molécule de liaison polyfonctionnelle, et dans laquelle la molécule de liaison polyfonctionnelle contient au moins une molécule de liaison choisie parmi le glutaraldéhyde, un agent de couplage au silane et un polymère à base d'éthylène glycol polyfonctionnel.

2. Lame de verre de contrôle selon la revendication 1, dans laquelle la substance de contrôle contient au moins une substance choisie parmi une protéine, un peptide, un oligomère d'acide aminé et un acide nucléique.

3. Lame de verre de contrôle selon la revendication 1, dans laquelle le matériau de piégeage de colorant contient au moins un matériau sélectionné parmi un polymère d'acide aminé, un polymère de sucre et un polymère synthétique.

4. Lame de verre de contrôle selon la revendication 3, dans laquelle
le polymère d'acide aminé contient au moins un polymère choisi parmi la gélatine, le collagène, l'albumine, l'acide polyglutamique et la polylysine,
le polymère de sucre contient au moins un polymère choisi parmi l'agarose, l'acide alginique, l'acide hyaluronique, le carraghénane, la pectine, la méthylcellulose et l'hydroxyéthylcellulose, et
le polymère synthétique contient au moins un polymère choisi parmi un polymère à base d'acide acrylique, un polymère à base d'acide méthacrylique, un polymère à base d'acrylamide, un polymère à base de méthacrylamide et un polymère à base d'éthylène glycol.

5. Lame de verre de contrôle selon la revendication 1, dans laquelle le point de contrôle présente une aire de 1 mm² ou plus et de 1 000 mm² ou moins.

6. Lame de verre de contrôle selon la revendication 1, dans laquelle le point de contrôle présente une épaisseur de 0,1 µm ou plus et de 1 000 µm ou moins.

7. Lame de verre de contrôle selon la revendication 1, dans laquelle le point de contrôle présente une épaisseur de 0,1 µm ou plus et de 200 µm ou moins.

8. Lame de verre de contrôle selon la revendication 1, comprenant une pluralité de points de contrôle.

9. Lame de verre de contrôle selon la revendication 8, dans laquelle une aire par point de contrôle est de 1 mm² ou plus et de 200 mm² ou moins.

10. Lame de verre de contrôle selon la revendication 1, dans laquelle la substance de contrôle contient une substance de contrôle marquée par fluorescence.

11. Lame de verre de contrôle selon l'une quelconque des revendications 1 à 10, dans laquelle le point de contrôle est sous une forme poreuse, une forme de gel gonflé ou une forme de xérogel.

12. Procédé de fabrication de la lame de verre de contrôle selon la revendication 1, le procédé comprenant les étapes consistant à :
préparer un mélange contenant une substance de contrôle et un matériau de piégeage de colorant ; et
former un point de contrôle contenant le mélange sur une lame de verre.

13. Procédé selon la revendication 12, dans lequel
le mélange est solidifié en une forme tubulaire, et
le procédé comprend l'étape consistant à former un point de contrôle sur une lame de verre en coupant le mélange en une forme de film mince et en fixant le mélange sous la forme de film mince à la lame de verre.

14. Procédé de fabrication de la lame de verre de contrôle selon la revendication 1, le procédé comprenant les étapes consistant à :
fixer une substance de contrôle sur une lame de verre ; et
former un point de contrôle sur la lame de verre en appliquant un matériau de piégeage de colorant sur la substance de contrôle fixée sur la lame de verre.
